# EUROPÄISCHE PATENTSCHRIFT

(11) **EP 1 605 872 B1**
(45) Veröffentlichungstag und Bekanntmachung des Hinweises auf die Patenterteilung: **30.09.2009**
(21) Anmeldenummer: 03816421.6
(22) Anmeldetag: 24.03.2003
(51) Int. Cl.: A61F 2/44

(54) **BANDSCHEIBEN- ODER ZWISCHENWIRBELPROTHESE**
VERTEBRAL DISC OR INTERVERTEBRAL DISC PROSTHESIS
PROTHESE DE DISQUE INTERVERTEBRAL OU PROTHESE INTERVERTEBRALE

(43) Veröffentlichungstag der Anmeldung: 21.12.2005
(73) Patentinhaber: Synthes GmbH, 4436 Oberdorf (CH)
(72) Erfinder: STUDER, Armin, CH-6330 Cham (CH); TRACHSEL, Jason, CH-2563 Ipsach (CH); WYMANN, Martin, CH-3097 Liebefeld (CH)
(74) Vertreter: Lusuardi, Werther
(86) Internationale Anmeldenummer: PCT/CH2003/000187
(87) Internationale Veröffentlichungsnummer: WO 2004/084774

(56) Entgegenhaltungen:
- EP-A- 0 346 269
- FR-A- 2 734 148
- US-A- 4 309 777
- US-A- 5 674 294
- US-A- 5 716 416
- US-A- 5 782 832

## Beschreibung

Die Erfindung bezieht sich auf eine Bandscheibenprothese oder Zwischenwirbelprothese gemäss dem Oberbegriff des Patentanspruchs 1. Solche Prothesen können als Nukleusersatz, flexibler Käfig (Cage) oder interne Bandscheibenprothese ausgebildet sein, welche von posterior (PLIF-Technik) einsetzbar sind. In Form von dynamischen Implantaten, können sie auch zwischen die Dornfortsätze benachbarter Wirbelkörper plaziert werden.

Aus der US-A-5 458 642 BEER ET AL. ist bereits eine Bandscheibenprothese bekannt mit einer oberen und einer unteren nierenförmigen Platte, welche an ihrer Peripherie (umlaufend um einen zentralen Kern) über eine Vielzahl von identischen Spiralfedern miteinander verbunden sind. Die Spiralfedern sind in regelmässigen Abständen zueinander angeordnet und erlauben eine dreidimensionale Bewegung der beiden Platten innerhalb gewisser Grenzen. Nachteilig bei dieser bekannten Bandscheibenprothese ist der Umstand, dass die Steifigkeit des Implantates in jeder radialen Richtung - bei gleichem Abstand vom Zentrum des Implantates - identisch ist, d.h. dass das Implantat eine symmetrische Steifigkeit aufweist.

Aus der US-A 4,309,777 PATIL ist eine Bandschreibenprothese mit mehreren symmetrischen Federelementen bekannt.

Aus der FR 2,734,148 A BIOMAT ist ein Implantat bekannt mit einer elliptischen Feder.

Schliesslich ist aus der EP-A 0346269 MECRON eine Bandscheibenprothese bekannt, welche ein einziges elastisches Mittel aufweist, welches aus viskoelastischem Material - umschlossen von einem Wellrohr - besteht und den gesamten Raum zwischen den beiden Endplatten einnimmt. Nachteilig bei diesem bekannten Implantat ist auch der Umstand, dass die elastischen Mittel fix mit den beiden Endplatten verbunden sind.

Hier will die Erfindung Abhilfe schaffen. Der Erfindung liegt die Aufgabe zugrunde, eine Bandscheibenprothese zu schaffen, welche eine asymmetrische Steifigkeit aufweist, die einfach an die Bedürfnisse des Patienten angepaßt werden kann. Dies hat den Vorteil, dass die Bandscheibenprothese konstruktiv auf ein physiologischeres Verhalten eingestellt werden kann, so dass beispielsweise bei einer Extension der Wirbelsäule nach hinten eine grössere Steifigkeit des Implantates vorhanden ist als bei einer lateralen Bewegung seitlich nach vorne.

Die Erfindung löst die gestellte Aufgabe mit einer Bandscheibenprothese, welche die Merkmale des Anspruchs 1 aufweist.

Die durch die Erfindung erreichten Vorteile sind im wesentlichen darin zu sehen, dass dank der erfindungsgemässen Bandscheibenprothese ein physiologischeres Verhalten bei Belastung der Wirbelsäule erreicht werden kann und insbesondere der Drehpunkt des Implantates durch die asymmetrische Steifigkeitsänderung und einfache Fixierung der elastischen Mittel an Appositionsplatten leichter gesteuert werden kann.

In einer bevorzugten Ausführungsform liegt die Anzahl der elastischen Mittel in einem Bereich von 4 bis 12, vorzugsweise von 6 bis 10.

In einer anderen Ausführungsform sind die elastischen Mittel untereinander identisch, aber radial ungleichmässig in den peripheren Bereichen der Bandscheibenprothese angeordnet. Dadurch ist der Vorteil erreichbar, dass durch eine variable Anzahl von identischen elastischen Mitteln pro Grad Peripherie oder alternativ durch eine unregelmässige (d.h. dichtere oder weniger dichte) Anordnung von identischen elastischen Elementen, sich die Steifigkeit der Bandscheibenprothese produktionsmässig wahlweise einstellen lässt, derart dass je nach radialer Richtung eine unterschiedliche Steifigkeit der Bandscheibenprothese resultiert, welche damit den anatomischen Gegebenheiten besser entspricht als dies bei herkömmlichen eine symmetrische Steifigkeit aufweisenden Bandscheibenprothesen der Fall ist.

In wiederum einer anderen Ausführungsform ist mindestens ein Teil der elastischen Mittel verschieden voneinander ausgestaltet, wobei die elastischen Mittel vorzugsweise radial gleichmässig in den peripheren Bereichen der Bandscheibenprothese angeordnet sind.

In einer weiteren Ausführungsform ist mindestens ein Teil der elastischen Mittel verschieden voneinander ausgestaltet, wobei die elastischen Mittel radial ungleichmässig in den peripheren Bereichen der Bandscheibenprothese angeordnet sind.

In wiederum einer weiteren Ausführungsform weist die Bandscheibenprothese mindestens in einem Teilbereich eines peripheren Bogens von 90° eine höhere Steifigkeit auf als im komplementären peripheren Bogen von 90°.

In einer anderen Ausführungsform besteht mindestens ein Teil der elastischen Mittel aus verschiedenen Materialien unterschiedlicher Steifigkeit.

Als Materialien eignen sich alle bekannten Implantatmaterialien metallischer und polymerer Natur. Das Implantat kann auch mit einer HAC-Beschichtung versehen sei.

Bevorzugte Materialien für das Implantat sind: Titan, Nitinol, Titanlegierungen und Stahl. Bevorzugte Material-Kombinationen sind folgende:
- für die Appositionsplatten: Titan/Titanlegierungen
- für die Zwischenplatten : Titan/Titanlegierungen
- für die Schrauben : Titan
- für die Ringe : Nitinol, Titan oder Stahl
   Die Geometrie und Oberfläche der Appositionsplatten sind zweckmässigerweise den natürlichen Endplatten der Wirbelkörper angepasst, wobei in verschiedenen Ausführungsformen die beiden Appositionsplatten kreisringförmig, rechteckförmig, nierenförmig, oval oder spiralförmig ausgebildet sein können.
   In wiederum einer weiteren Ausführungsform sind die elastischen Mittel als Ringe oder Teilringe ausgebildet, wobei die Anordnung der Ringebene der so ausgestalteten elastischen Mittel derart erfolgen kann, dass
- die Ringebene die Zentralachse der Bandscheibenprothese schneidet;
- die Ringebene die Zentralachse der Bandscheibenprothese nicht schneidet;
- die Ringebene im wesentlichen senkrecht zu den beiden Appositionsplatten verläuft; oder dass
- die Ringebene schräg zu den beiden Appositionsplatten steht.

In einer anderen Ausführungsform weist mindestens ein Teil der Ringe eine unterschiedliche Steifigkeit auf, wobei die Anordnung der Ringe vorzugsweise sequentiell mit zunehmender, beziehungsweise abnehmender Steifigkeit erfolgt.

In wiederum einer anderen Ausführungsform sind die Ringe peripher angeordnet, wodurch der Vorteil erreichbar ist, dass dadurch periphere Teilbereich mit höherer und solche mit tieferer Steifigkeit resultieren.

In verschiedenen Ausführungsformen können die elastischen Mittel aus folgender Gruppe ausgewählt werden: Spiralfedern, elastische Bälge, Kunststoffzylinder, Bänder, Drahtmaschengeflechte, Endlosfasern oder kunststoffbeschichtete Drähte. Damit sind gegenüber der Ausgestaltung der elastischen Mittel als Ringe folgende Vorteile erzielbar:
- erhöhte Flexibilität;
- einfacheres Produktions-Knowhow;
- einfacheres Handling; und
- ein viskoelastisches Verhalten der Bandscheibenprothese.

In einer weiteren Ausführungsform bestehen die elastischen Mittel aus einem Drahtseil, welches vorzugsweise als Unifilament ausgebildet ist.

In weiteren Ausführungsformen umfassen die elastischen Mittel mindestens ein Federelement, welches einem wie folgt ausgebildeten Federdraht besteht:
- der Federdraht weist Serpentinen auf; und/oder
- der Federdraht weist mindestens eine Schleife auf.
   In wiederum einer weiteren Ausführungsform umfasst die Bandscheibenprothese einen Kern aus einem Kunststoffmaterial. Damit sind die Vorteile erreichbar, dass die Bandscheibenprothese ein viskoelastisches Verhalten aufweist und eine bessere Dämpfung bei Bewegungen der angrenzenden Wirbelkörper erfolgt.
   In einer anderen Ausführungsform beträgt die Kompressibilität der Bandscheibenprothese an der Peripherie mindestens 0,7 mm, vorzugsweise mindestens 1,0 mm und höchstens 1,2 mm, vorzugsweise höchstens 3,5 mm.
   Die Verbindung der elastischen Mittel mit den Appositionsplatten kann form- oder kraftschlüssig ausgebildet sein.
   In wiederum einer anderen Ausführungsform schliessen die beiden Appositionsplatten untereinander einen Winkel von 10° bis 14° ein.
   Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Bandscheibenprothese;
Fig. 2 eine Seitenansicht der Bandscheibenprothese nach Fig. 1;
Fig. 3 eine Aufsicht auf die Bandscheibenprothese nach Fig. 1;
Fig. 4a eine Seitenansicht einer weiteren Ausführungsform der Bandscheibenprothese;
Fig. 4b eine Aufsicht auf die in Fig. 4a dargestellte Ausführungsform der Bandscheibenprothese ohne obere Apposltionsplatte;
Fig. 4c eine perspektivische Ansicht der in Fig. 4a dargestellten Ausführungsform der Bandscheibenprothese;
Fig. 4d eine perspektivische Ansicht der in den Fig. 4a bis 4c dargestellten Ausführungsform der Bandscheibenprothese ohne obere Appositionsplatte;
Fig. 5a eine Frontansicht einer weiteren Ausführungsform der Bandscheibenprothese ohne obere Appositionsplatte;
Fig. 5b eine Aufsicht auf die Fig. 5a dargestellten Ausführungsform der Bandscheibenprothese;
Fig. 5c eine Seitenansicht der in den Fig. 5a und 5b dargestellten Ausführungsform der Bandscheibenprothese;
Fig. 5d eine perspektivische Ansicht der in den Fig. 5a bis 5c dargestellten Ausführungsform der Bandscheibenprothese;
Fig. 6a eine Ansicht einer anderen Ausführungsform der Bandscheibenprothese;
Fig. 6b einen Schnitt B-B durch die in Fig. 6a dargestellte Ausführungsform der Bandscheibenprothese;
Fig. 6c eine perspektivische Ansicht der mit der unteren Appositionsplatte verbundenen Federelemente der in den Fig. 6a und 6b dargestellten Ausführungsform der Bandscheibenprothese ohne obere Appositionsplatte;
Fig. 6d eine perspektivische Ansicht der in den Fig. 6a bis 6c dargestellten Ausführungsform der Bandscheibenprothese;
Fig. 7a eine Aufsicht auf eine weitere Ausführungsform der Bandscheibenprothese;
Fig. 7b einen Schnitt B-B durch die in Fig. 7a dargestellte Ausführungsform der Bandscheibenprothese;
Fig. 7c eine Seitenansicht der in den Fig. 7a und 7b dargestellten Ausführungsform der Bandscheibenprothese;
Fig. 7d eine perspektivische Ansicht der in den Fig. 7a bis 7c dargestellten Ausführungsform der Bandscheibenprothese;
Fig. 8 eine schematische Darstellung zweier zwischen zwei Wirbelkörpern implantierten Bandscheibenprothesen wie in den Fig. 7a bis 7d dargestellt;
Fig. 9 eine Aufsicht parallel zur Wirbelsäulenlängsachse auf die Deckplatte eines Wirbelkörpers mit zwei Bandscheibenprothesen wie in den Fig. 7a bis 7d dargestellt;
Fig. 10 eine Ansicht von ventral zweier zwischen zwei Wirbelkörpern implantierten Bandscheibenprothesen wie in den Fig. 7a bis 7d dargestellt; und
Fig. 11 eine Ansicht einer weiteren Ausführungsform der elastischen Mittel der erfindungsgemässen Bandscheibenprothese.

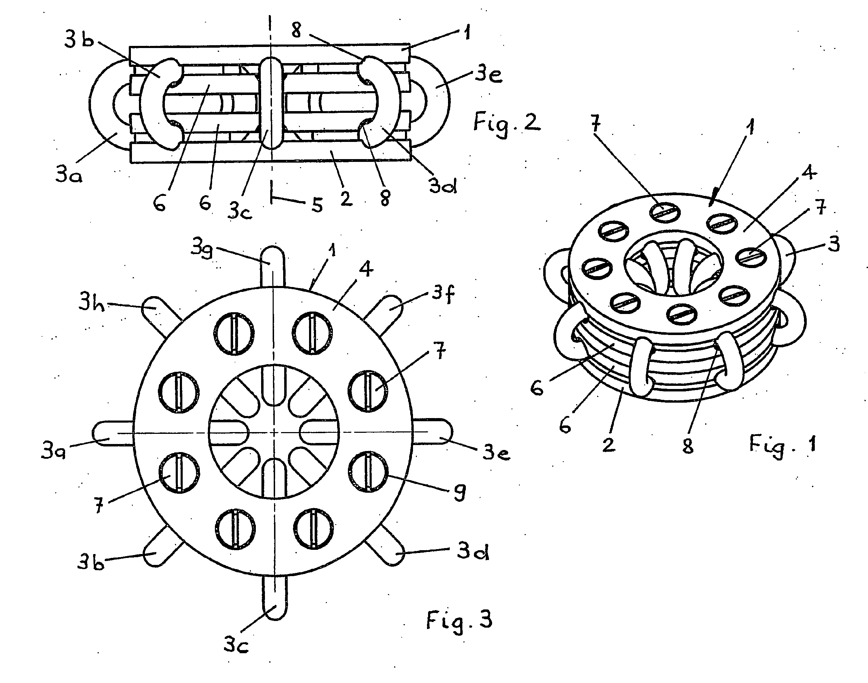

Die in den Fig. 1 bis 3 dargestellte Bandscheibenprothese besteht aus einer oberen, zur Anlage an die Grundplatte eines Wirbelkörpers geeigneten, kreisringförmigen Appositionsplatte 1, einer davon beabstandeten, zur Anlage an die Deckplatte eines Wirbelkörpers geeigneten unteren, kreisringförmigen Appositionsplatte 2, zwei zwischen den beiden Appositionsplatten 1,2 angeordneten, ebenfalls kreisringförmig ausgebildeten Zwischenplatten 6, wobei alle Platten entlang einer gemeinsamen Zentralachse 5 orthogonal dazu angeordnet sind.

Zwischen den beiden kreisringförmigen Appositionsplatten 1,2 sind insgesamt acht elastische Mittel 3 in Form von Ringen 3a - 3h angeordnet, welche radial zur Zentralachse 5 angeordnet sind und wobei deren Ringebene senkrecht zu den Appositionsplatten 1,2 liegt.

Damit die Bandscheibenprothese einen Zusammenhalt bekommt, ist die obere Appositionsplatte 1 mit der benachbarten Zwischenplatte 6 durch insgesamt acht Schrauben 7 und ebenso die untere Appositionsplatte 2 mit der benachbarten Zwischenplatte 6 durch insgesamt acht Schrauben 7 verbunden. Zu diesem Zweck weisen die Appositionsplatten 1,2 entsprechende Bohrungen 9 zur Aufnahme der Schraubenköpfe und die Zwischenplatten 6 entsprechende Gewindebohrungen zur Aufnahme der Schraubenschäfte auf. Die Schrauben 7 sind derart angeordnet, dass sie jeweils zwischen zwei benachbarten Ringen verlaufen.

Die einzelnen Ringe 3a - 3h werden dabei vom derart gebildeten oberen Plattenpaar 1,6 und vom unteren Plattenpaar 2,6 diametral gefangen, so dass die beiden Plattenpaare 1,6 und 2,6 über die Ringe 3a - 3h miteinander verbunden sind und dank der Elastizität der Ringe 3a - 3h aus der im unbelasteten Zustand bestehenden Parallelität - innerhalb gewisser Grenzen (Kompressibilität von ca. 1,0 bis 1,5 mm an der Peripherie) - gegeneinander geneigt werden können. Dadurch können die beiden Appositionsplatten (1,2) einen Winkel von etwa 12° untereinander einschliessen.

Um die Bauhöhe der Bandscheibenprothese gering zu halten, können die Appositionsplatten 1,2 und die Zwischenplatten 6 im Bereich des Durchgangs der Ringe 3a - 3h dem Profil der Ringe 3a - 3h entsprechende Ausnehmungen 8 aufweisen

Die peripher in regelmässigen Abständen von 45° angeordneten elastischen Ringe 3a - 3h weisen eine unterschiedliche Elastizität und Steifigkeit auf, welche durch unterschiedliche Materialien, unterschiedliche Geometrien der Ringe oder unterschiedliche Profile der Ringe (voll, hohl, rund, rechteckig) erreicht werden kann.
Dadurch ergibt sich im Bereich mit den Ringen 3b,3c und 3d eine höhere Steifigkeit als im Bereich mit den Ringen 3f, 3g und 3h. Bei Anwendung einer bestimmten Kraft senkrecht zur Appositionsplatte 1 über dem Bereich des Rings 3 c ergibt sich somit eine kleinere Kompression (Annäherung der beiden Appositionsplatten 1,2) als bei Anwendung der gleich grossen Kraft über dem Bereich des Rings 3g. Durch dieses asymmetrische Verhalten ist es möglich ein besseres physiologisches Verhalten der Bandscheibenprothese zu erreichen, indem bei einer Flexion der Wirbelsäule nach vorne, die Kompression der Ringe 3f, 3g und 3h grösser ausfällt als bei einer Flexion der Wirbelsäule nach hinten, welche die Ringe 3b, 3c und 3d komprimiert.

Die Federrate der einzelnen Ringe können zweckmässigerweise zwischen 50 % und 100 % variieren. Betragsmässig kann die Federrate zwischen 300 N/mm und 1'000 N/mm liegen.

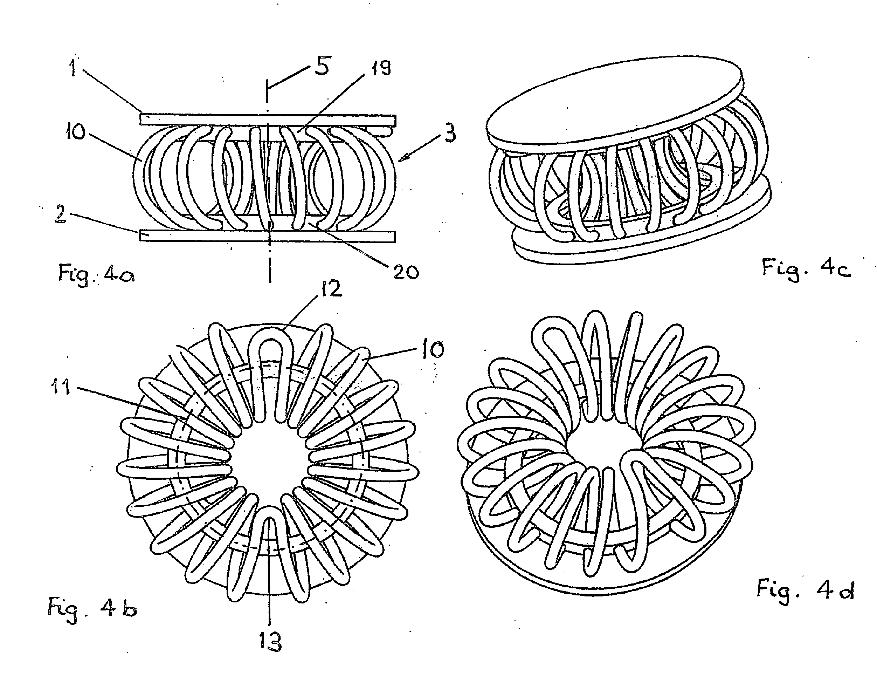

In den Fig. 4a bis 4d ist eine Ausführungsform dargestellt, bei welcher die elastischen Mittel 3 eine Schraubenfeder 10 umfassen, deren Längsachse 11 in einer zur Zentralachse 5 orthogonalen Ebene kreisförmig ausgebildet ist, so dass die Schraubenfeder 10 die Zentralachse 5 des Implantates mit einem Winkel von 360° umschliesst. Hier ist die Schraubenfeder 10 soweit gegen die Peripherie der kreisförmigen Appositionsplatten 1;2 nach aussen geschoben, dass sie die Peripherie der Appositionsplatten 1;2 leicht überragt. Die Schraubenfeder 10 weist zwei kreisbogenförmige Teile mit entgegengesetzter Steigung der Windungen des Federdrahtes auf. An den Verbindungsstellen der beiden Teile sind die Windungen der Schraubenfeder 10 durch je eine Schlaufe 12;13 miteinander verbunden. Die Schlaufen 12;13 sind hier an den gegen die obere Appositionsplatte 1 gerichteten Umfangssegmenten der Schraubenfeder 10 angeordnet. Durch die Ausgestaltung der Schraubenfeder 10 mit zwei kreisbogenförmigen Teilen, welche eine entgegengesetzte Steigung aufweisen, lässt sich die Torsionssteifigkeit des Implantates steuern. An den einander gegenüberliegenden Innenflächen der Appositionsplatten 1;2 sind konzentrisch zur Zentralachse 5 kreisringförmige Erhebungen 19;20 angebracht. Der Federdraht wird pro Windung je einmal durch Bohrungen in jeder der beiden kreisringförmigen Erhebungen 19;20 durchgeführt, so dass die Appositionsplatten 1;2 und die Schraubenfeder 10 zusammengehalten werden. Ferner sind die beiden Schlaufen 12;13 betreffend des Abstandes, welchen sie zwischen den beiden aneinander grenzenden Windungen des Federdrahtes einnehmen, verschieden ausgestaltet. Die Windungen der Schraubenfeder 10 weisen auf beiden kreisbogenförmigen Teilen der Schraubenfeder 10 eine konstante Steigung auf, so dass die Federkonstante des Implantates lediglich an den Verbindungsstellen der beiden kreisbogenförmigen Teile der Schraubenfeder 10 einen anderen Wert aufweist. Durch die Ausgestaltung der beiden Teile der Schraubenfeder 10 mit Windungen, welche eine entgegengesetzte Steigung aufweisen, ist erreichbar, dass die Torsionssteifigkeit des Implantates in beiden Drehrichtungen gleich gross ist.

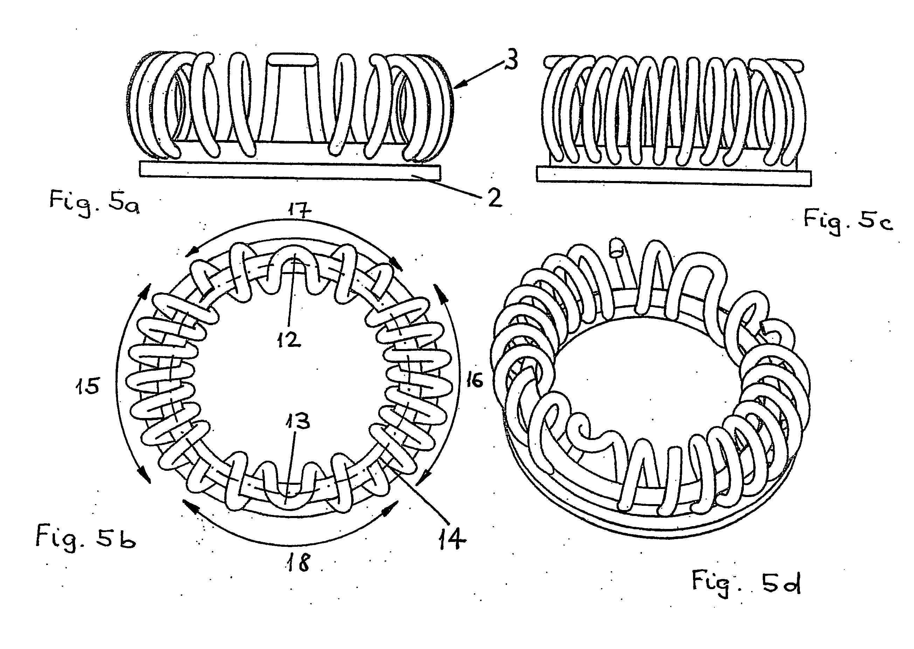

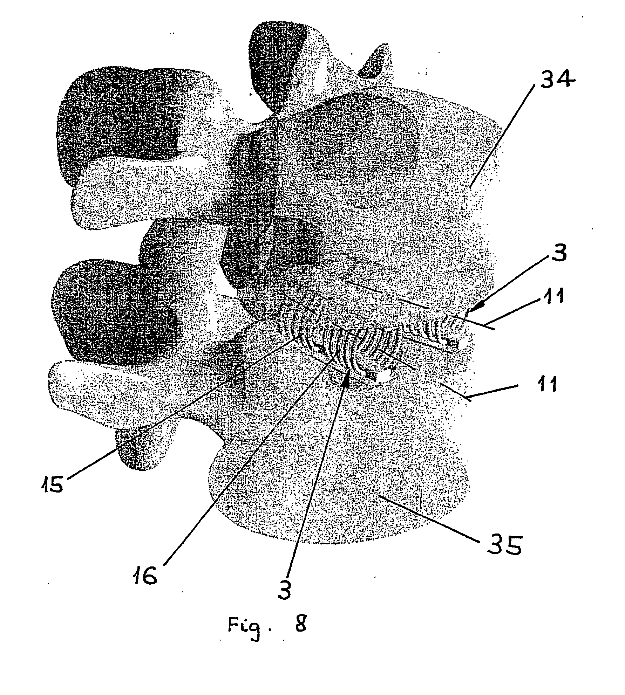

Die in den Fig. 5a bis 5d dargestellte Ausführungsform unterscheidet sich von der in den Fig. 4a bis 4d dargestellten Ausführungsform nur darin, dass die Appositionsplatten 1;2 (nur eine Appositionsplatte 2 gezeichnet) oval ausgestaltet sind und daher die elastischen Mittel 3 vier bogenförmige, auf einer ovalen Längsachse 14 angeordnete, aber getrennte Schraubenfederelemente 15;16;17;18 umfassen. Dabei sind je zwei einander diametral gegenüberliegende Schraubenfederelemente 15;16;17;18 spiegelsymmetrisch zueinander ausgestaltet, wobei zwei spiegelsymmetrisch angeordnete Schraubenfederelemente 15;16 Windungen mit entgegengesetzter Steigung aufweisen und die anderen zwei spiegelsymmetrisch angeordneten Schraubenfederelemente 17;18 jeweils in ihrer Mitte eine Schlaufe 12;13 aufweisen, wodurch sich der Drehsinn der Windungen ändert. Ferner sind die Steigungen der beiden Paare von spiegelsymmetrisch angeordneten Schraubenfederelemente 15;16;17;18 verschieden, so dass die Federkonstanten der elastischen Mittel 3 je nach Lage der Drehachse zwischen den beiden an die Aussenflächen der beiden Appositionsplatten 1;2 angrenzenden Wirbelkörper 34;35 (Fig. 8ff) verschieden sind.

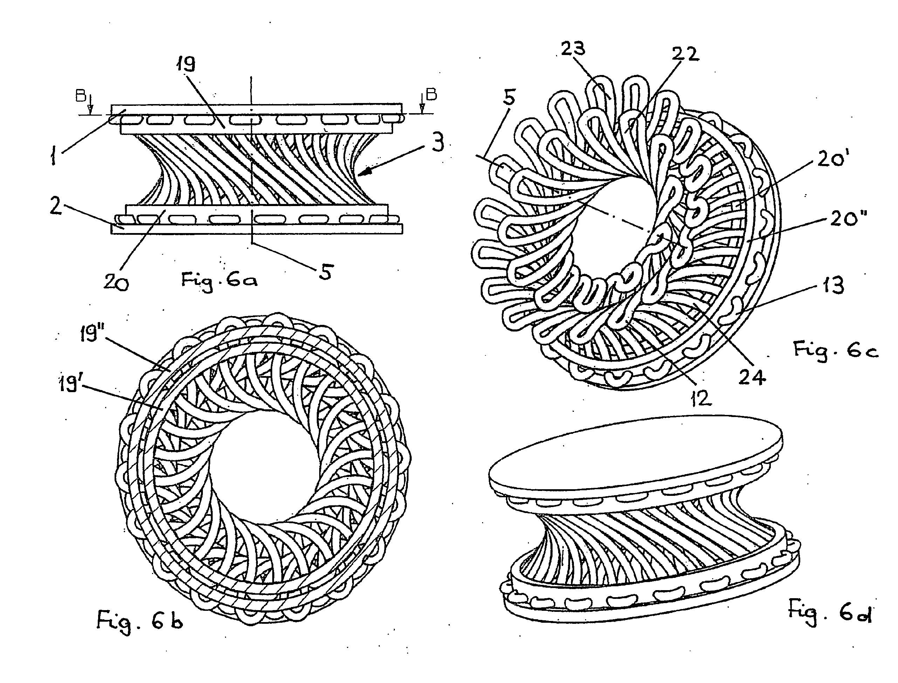

In den Fig. 6a bis 6d ist eine Ausführungsform dargestellt, deren Ausgestaltung der elastischen Mittel 3 sich gegenüber der in den Fig. 4 und 5 dargestellten Ausgestaltung nur darin unterscheidet, dass sie zwei zur Zentralachse 5 konzentrische Federelemente 22;23 umfasst, welche je einen mehrere Serpentinen 24 aufweisenden Federdraht 25 aufweisen. Die Federelemente 22;23 haben die Form von Torusteilflächen, wobei die Verbindungsstücke zwischen den Schlaufen 12;13 der Serpentine 24 schräg zu den Meridianen des Torus verlaufen. Dabei sind die Winkel zwischen den Meridianen des Torus und den Verbindungsstücken zwischen den Schlaufen 12;13 der Serpentinen 24 für die beiden Federelemente 22;23 entgegengesetzt mit gleichem Betrag. Ferner weist jede Appositionsplatte 1;2 zwei bezüglich der Zentralachse 5 konzentrische Erhebungen 19';19";20';20" auf. Je eine Schlaufe 12 einer Serpentine 24 ist analog zu den Ausführungsformen gemäss Fig. 4 durch zwei Bohrungen in einer der kreisringförmigen Erhebungen 19 an der oberen Appositionsplatte 1 geführt, während die andere Schlaufe 13 der Serpentine 24 durch zwei Bohrungen in einer der kreisringförmigen Erhebungen 20 an der unteren Appositionsplatte 2 geführt ist, so dass die beiden Appositionsplatten 1;2 und die elastischen Mittel 3 zusammengehalten werden. Dabei werden die Schlaufen 12;13 des inneren Federelementes 22 durch die Bohrungen in der inneren Erhebungen 19';20' und die Schlaufen 12;13 des äusseren Federelementes 23 durch die Bohrungen in den äusseren Erhebungen 19";20" geführt.

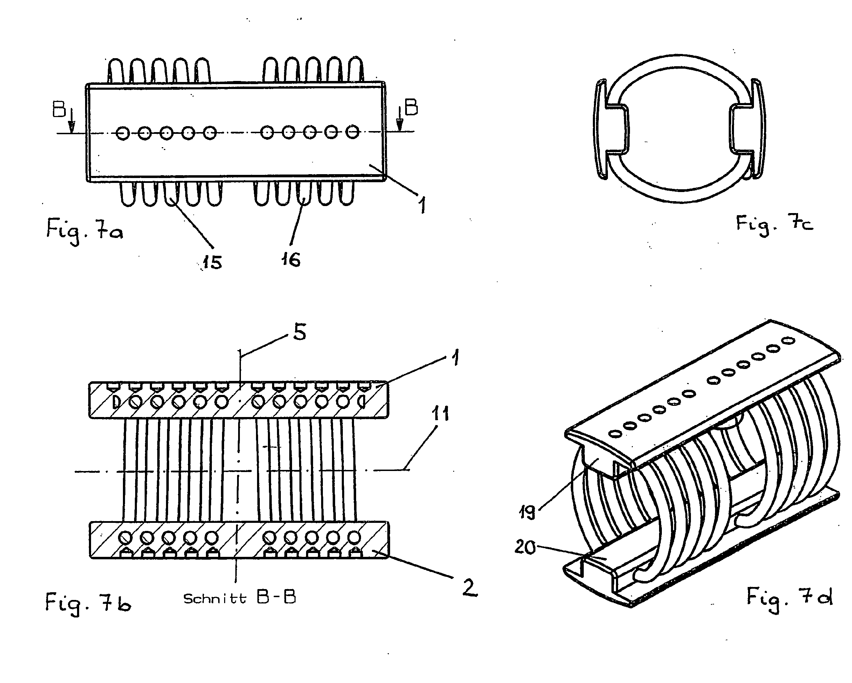

In den Fig. 7a bis 7d ist eine Ausführungsform dargestellt, welche eine obere und eine untere Appositionsplatte 1;2 mit quer zur Zentralachse 5 stehenden, rechteckförmigen Oberflächen umfasst. Die Längsachse 11 von zwei hintereinander zwischen den Appositionsplatten 1;2 angeordneten Schraubenfederelementen 15;16 ist parallel zu den langen Achsen der rechteckigen Appositionsplatten 1;2. Die zwei Schraubenfederelemente 15;16 weisen zueinander entgegengesetzte Steigungen ihrer Windungen auf. Ferner sind an den innen liegenden Oberflächen der zwei Appositionsflächen 1;2 zu den langen Achsen parallele Erhebungen 19;20 angeordnet, welche quer zu den langen Achsen verlaufende Bohrungen aufweisen. Die durch die Bohrungen durchgeführten Windungen der Schraubenfederelemente 15;16 halten somit die beiden Appositionsplatten 1;2 zusammen.

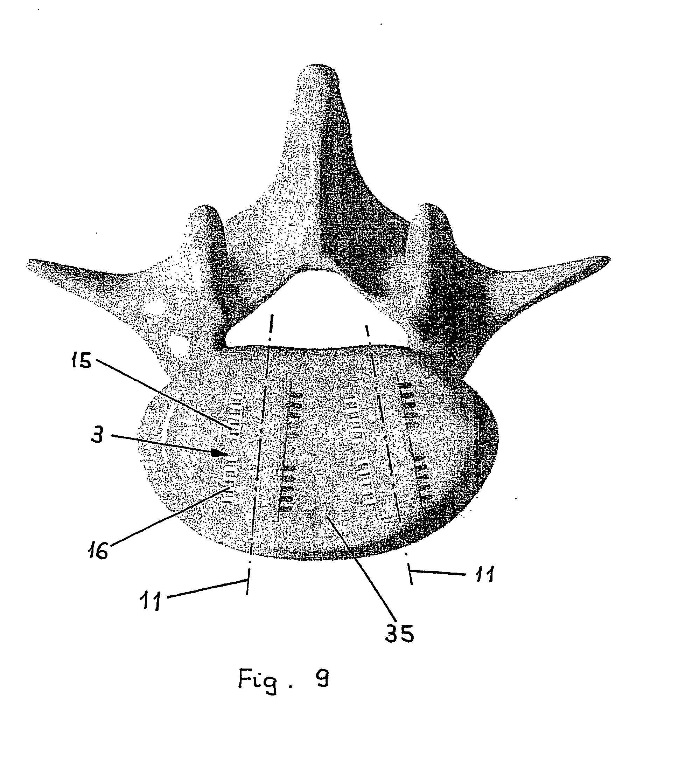

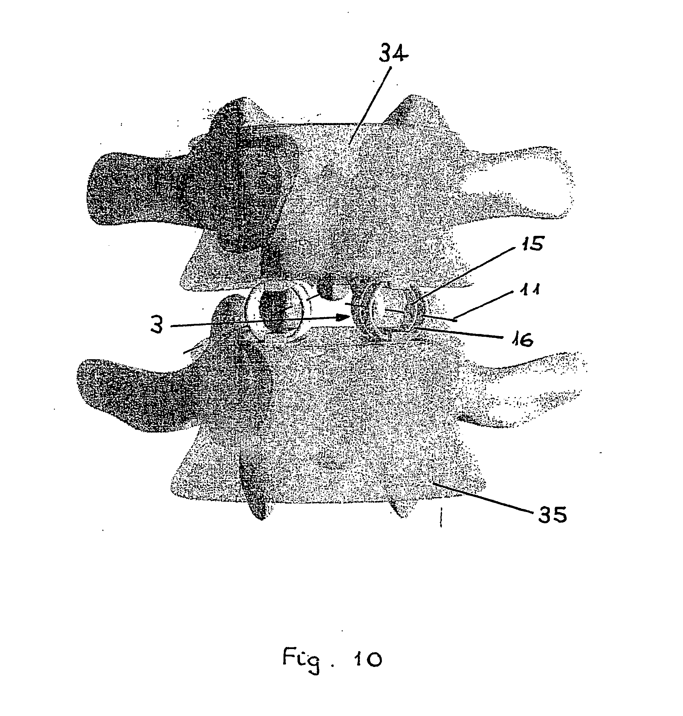

Die Anwendung zweier Bandscheibenprothesen wie in den Fig. 7a bis 7d dargestellt ist in den Fig. 8 bis 10 gezeigt. Die zwei Bandscheibenprothesen sind derart in den Zwischenwirbelraum zweier aneinander grenzenden Wirbelkörper 34;35 eingeführt, dass die Längsachsen 11 der Schraubenfederelemente 15;16 antero-posterior verläuft, wobei je eine Bandscheibenprothese lateral zur Wirbelsäulenlängsachse angeordnet ist. Durch diese Anordnung der Bandscheibenprothesen ergeben sich unterschiedliche Federraten der elastischen Mittel 3 für Flexion/Extension und laterale Beugungen der Wirbelsäule.

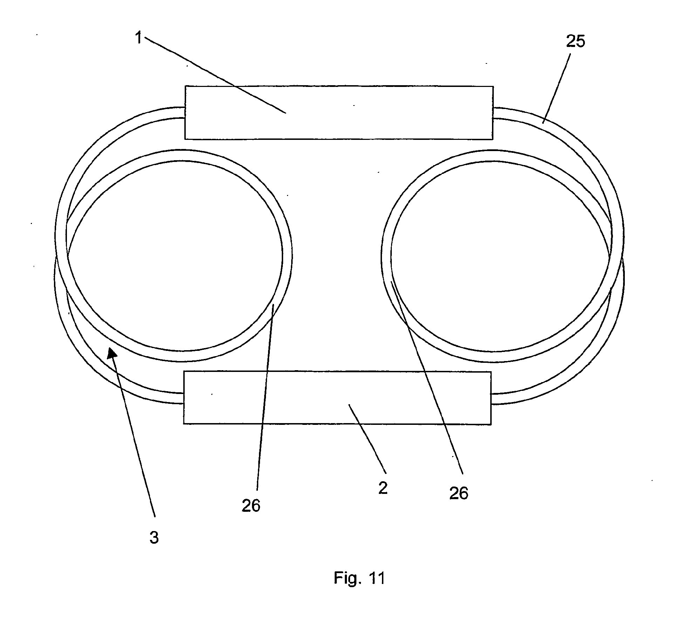

In Fig. 11 ist eine Ausführungsform der elastischen Mittel 3 dargestellt, welche einen in Schleifen 26 gewundenen Federdraht 25 umfasst. Die Schleifen 26 auf dem Federdraht 25 können einerseits derart ausgestaltet sein, dass analog zu den in Fig. 1 gezeigten Ringen die Schleifen 26 geschlossen sind und einzelne Federelemente bilden, welche in einer gewünschten Form zwischen den Appositionsplatten 1;2 verteilt sind. Andererseits können die Schleifen 26 analog zu der in Fig. 4 dargestellten Ausführungsform Windungen eines schraubenfederartigen Federelementes bilden. Die Verankerung des Federdrahtes 25 an den Appositionsplatten 1;2 kann nach einer der in den Fig. 1 bis 10 dargestellten Ausführungsform erfolgen. Ferner kann die Ausgestaltung der Appositionsplatten 1;2 sowie die Verteilung der elastischen Mittel 3 nach einer der in den Fig. 1 bis 10 dargestellten Ausführungsform erfolgen.

## Patentansprüche

1. Bandscheibenprothese oder Zwischenwirbelprothese mit
A) einer oberen, zur Anlage an die Grundplatte eines Wirbelkörpers geeigneten Appositionsplatte (1);
B) einer davon beabstandeten, zur Anlage an die Deckplatte eines Wirbelkörpers geeigneten unteren Appositionsplatte (2)
C) einer Mehrzahl von elastischen Mitteln (3), welche zwischen den beiden Appositionsplatten (1,2) in deren peripheren Bereichen (4) angeordnet sind, so dass die beiden Appositionsplatten (1,2) gegeneinander elastisch bewegbar sind;
D) einer Zentralachse (5) welche im Wesentlichen senkrecht auf den beiden Appositionsplatten (1,2) steht, wobei
E) die elastischen Mittel (3) derart ausgebildet, beziehungsweise angeordnet sind, dass die Bandscheibenprothese insgesamt eine asymmetrische Steifigkeit aufweist,
**dadurch gekennzeichnet, dass**
F) die elastischen Mittel (3) zu einer Einheit zusammengeschlossen sind, auf welche die beiden Appositionsplatten (1,2) aufschiebbar oder aufklickbar sind.

2. Bandscheibenprothese nach Anspruch 1, **dadurch gekennzeichnet, dass** die Anzahl der elastischen Mittel (3) im Bereich von 4 - 12, vorzugsweise von 6 - 10 liegt.

3. Bandscheibenprothese nach Anspruch 1 oder 2, **dadurch gekennzeichnet, dass** die elastischen Mittel (3) untereinander identisch sind aber radial ungleichmässig in den peripheren Bereichen (4) angeordneten sind.

4. Bandscheibenprothese nach Anspruch 1 oder 2, **dadurch gekennzeichnet, dass** mindestens ein Teil der elastischen Mittel (3) verschieden sind und vorzugsweise radial gleichmässig in den peripheren Bereichen (4) angeordneten sind.

5. Bandscheibenprothese nach Anspruch 1 oder 2, **dadurch gekennzeichnet, dass** mindestens ein Teil der elastischen Mittel (3) verschieden sind und radial ungleichmässig in den peripheren Bereichen (4) angeordneten sind.

6. Bandscheibenprothese nach einem der Ansprüche 1 bis 5, **dadurch gekennzeichnet, dass** sie mindestens in einem Teilbereich eines peripheren Bogen von 90° eine höhere Steifigkeit aufweist als im komplementären peripheren Bogen von 90°.

7. Bandscheibenprothese nach einem der Ansprüche 1 bis 6, **dadurch gekennzeichnet, dass** mindestens ein Teil der elastischen Mittel (3) aus verschiedenen Materialien unterschiedlicher Steifigkeit bestehen.

8. Bandscheibenprothese nach einem der Ansprüche 1 bis 7, **dadurch gekennzeichnet, dass** die beiden Appositionsplatten (1,2) kreisringförmig ausgebildet sind.

9. Bandscheibenprothese nach einem der Ansprüche 1 bis 8, **dadurch gekennzeichnet, dass** die elastischen Mittel (3) Ringe oder Teilringe sind.

10. Bandscheibenprothese nach Anspruch 9, **dadurch gekennzeichnet, dass** die Ringebene der als Ringe oder Teilringe ausgebildeten elastischen Mittel (3) die Zentralachse (5) schneidet.

11. Bandscheibenprothese nach Anspruch 9, **dadurch gekennzeichnet, dass** die Ringebene der als Ringe oder Teilringe ausgebildeten elastischen Mittel (3) die Zentralachse (5) nicht schneidet.

12. Bandscheibenprothese nach einem der Ansprüche 9 bis 11, **dadurch gekennzeichnet, dass** die Ringebene im Wesentlichen senkrecht zu den beiden Appositionsplatten (1,2) verläuft.

13. Bandscheibenprothese nach einem der Ansprüche 9 bis 11, **dadurch gekennzeichnet, dass** die Ringebene schräg zu den beiden Appositionsplatten (1,2) verläuft.

14. Bandscheibenprothese nach einem der Ansprüche 9 bis 13, **dadurch gekennzeichnet, dass** mindestens ein Teil der Ringe eine unterschiedliche Steifigkeit aufweist und vorzugsweise sequentiell mit zunehmender, beziehungsweise abnehmender Steifigkeit angeordnet sind.

15. Bandscheibenprothese nach Anspruch 14, **dadurch gekennzeichnet, dass** die Ringe peripher angeordnet sind.

16. Bandscheibenprothese nach einem der Ansprüche 1 bis 8, **dadurch gekennzeichnet, dass** die elastischen Mittel (3) aus folgender Gruppe ausgewählt sind: Spiralfedern, elastische Bälge, Kunststoffzylinder, Bänder, Drahtmaschengeflechte, Endlosfasern und kunstoffbeschichtetete Drähte

17. Bandscheibenprothese nach einem der Ansprüche 1 bis 8, **dadurch gekennzeichnet, dass** die elastischen Mittel (3) aus einem Unifilament ausgebildet ist.

18. Bandscheibenprothese nach einem der Ansprüche 1 bis 8, **dadurch gekennzeichnet, dass** die elastischen Mittel (3) mindestens ein Federelement (22;23) umfassen, welches aus einem Serpentinen (24) aufweisenden Federdraht besteht.

19. Bandscheibenprothese nach einem der Ansprüche 1 bis 8, **dadurch gekennzeichnet, dass** die elastischen Mittel (2) mindestens ein Federelement umfassen, welches aus einem mindestens eine Schleife (26) aufweisenden Federdraht (25) besteht.

20. Bandscheibenprothese nach einem der Ansprüche 1 bis 19, **dadurch gekennzeichnet, dass** sie einen Kern aus einem Kunststoff-Material aufweist.

21. Bandscheibenprothese nach einem der Ansprüche 1 bis 20, **dadurch gekennzeichnet, dass** die beiden Appositionsplatten (1,2) nierenförmig oder oval oder spiralförmig ausgebildet sind.

22. Bandscheibenprothese nach einem der Ansprüche 1 bis 21, **dadurch gekennzeichnet, dass** ihre Kompressibilität an der Peripherie mindestens 0,7 mm, vorzugsweise mindestens 1,0 mm beträgt.

23. Bandscheibenprothese nach einem der Ansprüche 1 bis 22, **dadurch gekennzeichnet, dass** ihre Kompressibilität an der Peripherie höchstens 1,2 mm, vorzugsweise höchstens 3,5 mm beträgt.

24. Bandscheibenprothese nach einem der Ansprüche 1 bis 23, **dadurch gekennzeichnet, dass** die elastischen Mittel (3) mit beiden Appositionsplatten (1,2) form- oder kraftschlüssig verbunden sind.

25. Bandscheibenprothese nach einem der Ansprüche 1 bis 24, **dadurch gekennzeichnet, dass** die beiden Appositionsplatten (1,2) einen Winkel von 10° bis 14° untereinander einschliessen.

26. Bandscheibenprothese nach einem der Ansprüche 1 bis 25, **dadurch gekennzeichnet, dass** die elastischen Mittel (3) aus einem Drahtseil bestehen.

## Claims

1. Intervertebral disk prosthesis or intervertebral prosthesis comprising
(A) an upper apposition plate (1) appropriate to rest against the base plate of a vertebra,
(B) a lower apposition plate (2) spaced apart from the upper apposition plate (1) and suitable to rest against the upper plate of a vertebra,
(C) a plurality of elastic means (3) mounted between the two apposition plates (1, 2) in their peripheral zones (4) in a manner that the two apposition plates (1, 2) are displaceable in mutually resilient manner, and
(D) a central axis (5) which runs essentially perpendicularly to the two apposition plates (1,2), whereby
(E) the elastic means (3) are designed respectively configured in such a manner that the intervertebral disk prosthesis as a whole exhibits asymmetrical stiffness,
**characterized in that**
F) the elastic means (3) are combined into one unit on which the two apposition plates (1,2) may be slipped onto or snapped onto.

2. Intervertebral disk prosthesis as claimed in claim 1, **characterized in that** the number of the elastic means (3) is in the range of 4 to 12, preferably 6 to 10.

3. Intervertebral disk prosthesis as claimed in either of claims 1 and 2, **characterized in that** the elastic means (3) are mutually identical but are configured radially unevenly in the peripheral zones (4).

4. Intervertebral disk prosthesis as claimed in either of claims 1 and 2, **characterized in that** at least a portion of the elastic means (3) is different and is configured preferably in radially uniform manner in the peripheral zones (4).

5. Intervertebral disk prosthesis as claimed in either of claims 1 and 2, **characterized in that** at least a portion of the elastic means (3) is different and is configured in radially uneven manner in the peripheral zones (4).

6. Intervertebral disk prosthesis in one of claims 1 through 5, **characterized in that** in at least one segment of it exhibits higher stiffness in at least one peripheral arc of 90° than in the complementary peripheral arc of 90°.

7. Intervertebral disk prosthesis as claimed in one of claims 1 through 6, **characterized in that** at least a portion of the elastic means (3) is made of different materials exhibiting different stiffnesses.

8. Intervertebral disk prosthesis as claimed in one of claims 1 through 7, **characterized in that** the two apposition plates (1, 2) are circular/annular.

9. Intervertebral disk prosthesis as claimed in one of claims 1 through 8, **characterized in that** the elastic means (3) are rings or partial rings.

10. Intervertebral disk prosthesis as claimed in claim 9, **characterized in that** ring plane of the elastic means (3) designed as rings or partial rings intersects the central axis (5).

11. Intervertebral disk prosthesis as claimed in claim 9, **characterized in that** the ring plane of the elastic means (3) designed as rings or partial rings do not intersect the central axis (5).

12. Intervertebral disk prosthesis as claimed in one of claims 9 through 11, **characterized in that** the ring plane runs substantially perpendicularly to the two apposition planes (1, 2).

13. Intervertebral disk prosthesis as claimed in one of claims 9 through 11, **characterized in that** the ring plane runs obliquely to the two apposition plates (1, 2).

14. Intervertebral disk prosthesis as claimed in one of claims 9 through 13, **characterized in that** at least one portion of the rings exhibits a different stiffness and is configured preferably in a sequence of increasing respectively decreasing stiffness.

15. Intervertebral disk prosthesis as claimed in claim 14, **characterized in that** the rings are arrayed peripherally.

16. Intervertebral disk prosthesis as claimed in one of claims 1 through 8, **characterized in that** the elastic means (3) are selected from the following group: helical springs, resilient bellows, plastic cylinders, tapes/bands, wire mesh lattices, endless fibers sand plastic-coated wires.

17. Intervertebral disk prosthesis as claimed in one of claims 1 through 8, **characterized in that** the elastic means (3) are a wire rope, preferably a unifilament.

18. Intervertebral disk prosthesis as claimed in one of claims 1 through 8, **characterized in that** the elastic means (3) include at least one spring element (22; 23) consisting of a spring wire exhibiting serpentines (24).

19. Intervertebral disk prosthesis as claimed in one of claims 1 through 8, **characterized in that** the elastic means (3) include at least one spring element consisting of a spring wire exhibiting at least one loop (26).

20. Intervertebral disk prosthesis as claimed in one of claims 1 through 19, **characterized in that** it comprises a plastic core.

21. Intervertebral disk prosthesis as claimed in one of claims 1 through 20, **characterized in that** the two apposition plates (1, 2) are kidney-shaped, or oval, or spiral.

22. Intervertebral disk prosthesis as claimed in one of claims 1 through 21, **characterized in that** its compressibility at the periphery is at least 0.7 mm, preferably at least 1.0 mm.

23. Intervertebral disk prosthesis as claimed in one of claims 1 through 22, **characterized** its compressibility at the periphery is at most 1.2 mm, preferably at most 3.5 mm.

24. Intervertebral disk prosthesis as claimed in one of claims 1 through 23, **characterized** the elastic means (3) are connected to the two apposition plates (1, 2) in geometrically locking, i.e. positive manner or in frictionally locking manner.

25. Intervertebral disk prosthesis as claimed in one of claims 1 through 24, **characterized in that** the two apposition plates (1, 2) subtend between them an angle of 10 to 14°.

26. Intervertebral disk prosthesis as claimed in one of claims 1 through 25, **characterized in that** the elastic means (3) are wire ropes.

## Revendications

1. Prothèse de disque intervertébral ou prothèse intervertébrale comprenant :
A) une plaque d'apposition supérieure (1) appropriée pour venir en appui sur la plaque de base d'un corps vertébral ;
B) une plaque d'apposition inférieure (2) espacée de la plaque d'apposition supérieure et appropriée pour venir en appui sur la plaque de recouvrement d'un corps vertébral ;
C) une pluralité de moyens élastiques (3) qui sont disposés entre les deux plaques d'apposition (1, 2), dans leurs zones périphériques (4), de sorte que les deux plaques d'apposition (1, 2) sont mobiles élastiquement l'une par rapport à l'autre ;
D) un axe central (5) qui est sensiblement perpendiculaire aux deux plaques d'apposition (1, 2),
dans laquelle
E) les moyens élastiques (3) sont configurés ou disposés de manière telle, que la prothèse de disque intervertébral présente, dans l'ensemble, une rigidité asymétrique,
**caractérisée en ce que**
F) les moyens élastiques (3) sont refermés pour former un ensemble sur lequel les deux plaques d'apposition (1, 2) peuvent être introduites en coulissant ou en cliquant.

2. Prothèse de disque intervertébral selon la revendication 1, **caractérisée en ce que** le nombre de moyens élastiques (3) est dans la plage comprise entre 4 et 12, de préférence entre 6 et 10.

3. Prothèse de disque intervertébral selon la revendication 1 ou 2, **caractérisée en ce que** les moyens élastiques (3) sont identiques entre eux mais sont disposés dans les zones périphériques (4), de façon non uniforme dans le sens radial.

4. Prothèse de disque intervertébral selon la revendication 1 ou 2, **caractérisée en ce qu'**au moins une partie des moyens élastiques (3) est différente et est disposée dans les zones périphériques (4), de préférence de façon uniforme dans le sens radial.

5. Prothèse de disque intervertébral selon la revendication 1 ou 2, **caractérisée en ce qu'**au moins une partie des moyens élastiques (3) est différente et est disposée dans les zones périphériques (4), de façon non uniforme dans le sens radial.

6. Prothèse de disque intervertébral selon l'une des revendications 1 à 5, **caractérisée en ce qu'**elle présente, au moins dans une zone partielle d'un arc périphérique de 90°, une rigidité plus grande que dans l'arc périphérique complémentaire de 90°.

7. Prothèse de disque intervertébral selon l'une des revendications 1 à 6, **caractérisée en ce qu'**au moins une partie des moyens élastiques (3) se compose de matériaux différents, de rigidité variable.

8. Prothèse de disque intervertébral selon l'une des revendications 1 à 7, **caractérisée en ce que** les deux plaques d'apposition (1, 2) sont configurées en forme d'anneau de cercle.

9. Prothèse de disque intervertébral selon l'une des revendications 1 à 8, **caractérisée en ce que** les moyens élastiques (3) sont des anneaux ou des anneaux partiels.

10. Prothèse de disque intervertébral selon la revendication 9, **caractérisée en ce que** le plan annulaire des moyens élastiques (3) configurés comme des anneaux ou des anneaux partiels coupe l'axe central (5).

11. Prothèse de disque intervertébral selon la revendication 9, **caractérisée en ce que** le plan annulaire des moyens élastiques (3) configurés comme des anneaux ou des anneaux partiels ne coupe pas l'axe central (5).

12. Prothèse de disque intervertébral selon l'une des revendications 9 à 11, **caractérisée en ce que** le plan annulaire s'étend sensiblement de façon perpendiculaire aux deux plaques d'apposition (1, 2).

13. Prothèse de disque intervertébral selon l'une des revendications 9 à 11, **caractérisée en ce que** le plan annulaire s'étend en oblique par rapport aux deux plaques d'apposition (1, 2).

14. Prothèse de disque intervertébral selon l'une des revendications 9 à 13, **caractérisée en ce qu'**au moins une partie des anneaux présente une rigidité variable et est disposée en ayant une rigidité augmentant ou diminuant, de préférence successivement.

15. Prothèse de disque intervertébral selon la revendication 14, **caractérisée en ce que** les anneaux sont disposés de façon périphérique.

16. Prothèse de disque intervertébral selon l'une des revendications 1 à 8, **caractérisée en ce que** les moyens élastiques (3) sont sélectionnés parmi le groupe suivant: ressorts à spirale, soufflets élastiques, cylindres en matière plastique, bandes, treillis métalliques, fibres continues et fils métalliques recouverts de matière plastique.

17. Prothèse de disque intervertébral selon l'une des revendications 1 à 8, **caractérisée en ce que** les moyens élastiques (3) sont configurés à partir d'un filament unitaire.

18. Prothèse de disque intervertébral selon l'une des revendications 1 à 8, **caractérisée en ce que** les moyens élastiques (3) comprennent au moins un élément faisant ressort (22, 23) qui se compose d'un fil d'acier pour ressort comportant un serpentin (24).

19. Prothèse de disque intervertébral selon l'une des revendications 1 à 8, **caractérisée en ce que** les moyens élastiques (3) comprennent au moins un élément à ressort qui se compose d'un fil d'acier pour ressort (25) comportant au moins une boucle (26).

20. Prothèse de disque intervertébral selon l'une des revendications 1 à 19, **caractérisée en ce qu'**elle comporte un noyau en matière plastique.

21. Prothèse de disque intervertébral selon l'une des revendications 1 à 20, **caractérisée en ce que** les deux plaques d'apposition (1, 2) sont configurées de façon réniforme ou ovale ou en forme de spirale.

22. Prothèse de disque intervertébral selon l'une des revendications 1 à 21, **caractérisée en ce que** sa compressibilité, à la périphérie, est au moins de 0,7 mm, de préférence au moins de 1,0 mm.

23. Prothèse de disque intervertébral selon l'une des revendications 1 à 22, **caractérisée en ce que** sa compressibilité, à la périphérie, est au maximum de 1,2 mm, de préférence au maximum de 3,5 mm.

24. Prothèse de disque intervertébral selon l'une des revendications 1 à 23, **caractérisée en ce que** les moyens élastiques (3) sont reliés aux deux plaques d'apposition (1, 2), par complémentarité de forme ou à force.

25. Prothèse de disque intervertébral selon l'une des revendications 1 à 24, **caractérisée en ce que** les deux plaques d'apposition (1, 2) forment entre elles un angle de 10° à 14°.

26. Prothèse de disque intervertébral selon l'une des revendications 1 à 25, **caractérisée en ce que** les moyens élastiques (3) se composent d'un câble métallique.
